# EUROPEAN PATENT APPLICATION

(11) **EP 4 239 637 A1**
(43) Date of publication of application: **06.09.2023**
(21) Application number: 22176166.1
(22) Date of filing: 30.05.2022
(51) Int. Cl.: G16B 10/00, G16B 40/20, G16B 50/10

(54) **CONSTRUCTION METHOD OF RIBOSOMAL RNA DATABASE**

(30) Priority: 03.03.2022 TW 111107822
(71) Applicant: Acer Incorporated, New Taipei City 221 (TW); Acer Medical Inc., 22181 New Taipei City (TW); Chang Gung Memorial Hospital, Keelung, Anle Dist. 20401 Keelung City (TW); National Health Research Institutes, Zhunan Town 350 Miaoli County (TW)
(72) Inventor: CHAN, Yun-Hsuan, 221 New Taipei City (TW); WU, I-Wen, 20401 Keelung City (TW); LIN, Chieh Hua, 350 Miaoli County (TW); HSU, Yin-Hsong, 22181 NEW TAIPEI CITY (TW); YEH, Chi-Hsiao, 20401 Keelung City (TW); LIAO, Yu-Chieh, 350 Miaoli County (TW); TSAI, Tsung-Hsien, 221 New Taipei City (TW)
(74) Representative: 2K Patentanwälte Blasberg Kewitz & Reichel

(57) **Abstract**

A construction method of ribosomal RNA database is provided, including the following steps: selecting a source of nucleic acid sequence database; performing normalization and homogenization on species classification rules; using AI technology for normalized classification and correction; selecting the kingdom to which the sequence species belongs; filtering out redundant sequences and sequences with inconsistent lengths; setting a threshold for unknown bases other than A, T, C or G, and excluding unknown bases that exceed the threshold; and excluding sequences with insufficient classification information.

## Description

### BACKGROUND

### Field of the Disclosure

The present disclosure relates to a method for constructing a database, and more particularly, to a method for constructing a ribosomal RNA database.

### Description of Related Art

In recent years, the rapid development of high-throughput gene sequencing technology has expedited the research on microbial organisms, and the amount of microbial sequence data has been considerably expanded. Specifically, ribosomal RNA genes are often used as genetic marker of microorganism for species classification to be performed, thereby inferring the regulatory functions played by bacteria in the human body. Among the ribosomal RNA genes, the 16S of prokaryotes (including archaea and bacteria) and the 18S small subunit rRNA (SSU rRNA) of eukaryotes are the most important genetic markers. In addition, 23S/28S large subunit rRNA (LSU rRNA) is analyzed together with adjacent SSU rRNA, so that more species classification information may be obtained.

In the large database of microorganism, the correctness and integrity of the data might directly or indirectly affect the subsequent analysis and prediction results of various microbial phases. At present, the main sequence databases may be divided into two categories: native repository database and value-added database. The native repository database is mainly the International Nucleotide Sequence Database Collaboration

(INSDC). Members of the database include NCBI, EMBL, and DDBJ, which are mainly uploaded by researchers to provide sequences and related species classification information. This type of database has the largest number of sequences, but there is a lot of data noise and too much invalid information is involved. Value-added databases, such as SILVA, EzBioCloud, Greengenes, mainly include sequences in the INSDC database, and then perform redundant sequence exclusion and high-similarity sequence aggregation. Unknown sequences are subjected to sequence comparison or evolutionary tree analysis to give name or specific number to species. The above method may further reduce the amount of data in the native repository database. However, because of the inconsistency in the processing of unknown sequences, the classification information of the sequences might be erroneous.

The two types of databases mentioned above are lack of normalization and homogenization of classification information. In subsequent related microbiological analysis, the results of prediction are often affected by misplacement of classification information or minor discrepancies of characters. Therefore, developing a method for constructing a ribosomal RNA database capable of increasing the accuracy of the data and improving the prediction accuracy is an important issue for current research.

### SUMMARY OF THE DISCLOSURE

The present disclosure provides a method for constructing a ribosomal RNA database, which may increase the accuracy of the data to improve the prediction accuracy, and may be applied to various subsequent analysis methods to maintain the consistency and accuracy of results.

In the disclosure, a construction method of ribosomal RNA database includes the following steps: selecting a source of nucleic acid sequence database; performing normalization and homogenization on species classification rules; using AI technology for normalized classification and naming; selecting the kingdom to which the sequence species belongs; filtering out redundant sequences and sequences with inconsistent lengths; setting a threshold for unknown bases other than A, T, C or G, and excluding unknown bases that exceed the threshold; and excluding sequences with insufficient classification information.

In an embodiment of the present disclosure, the nucleic acid sequence database includes a native repository database or a value-added database.

In an embodiment of the present disclosure, the ribosomal RNA database includes a 16S rRNA gene database.

In an embodiment of the present disclosure, a seventh-order nomenclature is used for normalization to form a hierarchy relation table. The hierarchies defined in the seventh-order nomenclature include kingdom, phylum, class, order, family, genus, and species.

In an embodiment of the present disclosure, the method for homogenization includes finding out information of other hierarchy in the classification hierarchy relation table based on species names in the nucleic acid sequence database, or using the serial number as a search target for comparison with a database that stores serial numbers based on the serial number of species in the nucleic acid sequence database. After the species name of the serial number is found, the information of other hierarchy may be found from the classification hierarchy relation table.

In an embodiment of the present disclosure, the step of using AI technology to perform normalized classification and naming includes performing comparison according to the species hierarchy, so as to confirm that there is no repetition in the sequence classification information.

In an embodiment of the present disclosure, the step of selecting the kingdom to which the sequence species belongs includes selecting sequences belonging to the kingdom of Archaea and the kingdom of Bacteria directed at the 16S rRNA gene database, and excluding other kingdoms or sequences where the kingdom name is mistakenly named as Archaea or Bacteria.

In an embodiment of the present disclosure, in the 16S rRNA gene database, when the sequence contains the same species sequence with 100% identical conditions, the sequence is a redundant sequence.

In an embodiment of the present disclosure, in the 16S rRNA gene database, the sequences with inconsistent lengths are those that are shorter than 1200 bases or longer than 1800 bases in length.

Based on the above, the construction method of ribosomal RNA database of the present disclosure includes retrieving high-quality sequence data from the value-added database, and normalizing and homogenizing the classification information. In this way, not only that the sequences with high representativeness may be effectively filtered out, but also the amount of data may be reduced while the coverage of species at all hierarchies of classification may be increased. The database constructed through this process may be applied to various subsequent analysis methods to maintain the consistency and accuracy of results.

### BRIEF DESCRIPTION OF THE DRAWINGS

FIG. 1 is a schematic flowchart of a method for constructing a ribosomal RNA database according to an embodiment of the present disclosure.
FIG. 2 and FIG. 3 are schematic diagrams of a homogenization method in the construction method of a ribosomal RNA database according to an embodiment of the present disclosure.
FIG. 4 is a schematic diagram of performing normalized classification and naming using AI technology in the method for constructing a ribosomal RNA database according to an embodiment of the present disclosure.
FIG. 5 is a schematic diagram of excluding sequences with insufficient classification information in the method for constructing a ribosomal RNA database according to an embodiment of the present disclosure.

### DESCRIPTION OF EMBODIMENTS

As used herein, a range defined by "one value to another value" is a general description that avoids listing all the values in a range in the specification. Therefore, the recitation of a particular numerical range includes any numerical value within the numerical range and a smaller numerical range defined by any numerical value within the numerical range, and such recitation is equivalent to explicitly describing said any numerical value and said smaller numerical value in the specification.

The following examples will be described in detail in conjunction with the accompanying drawings, but the provided examples are not intended to limit the scope of the present disclosure.

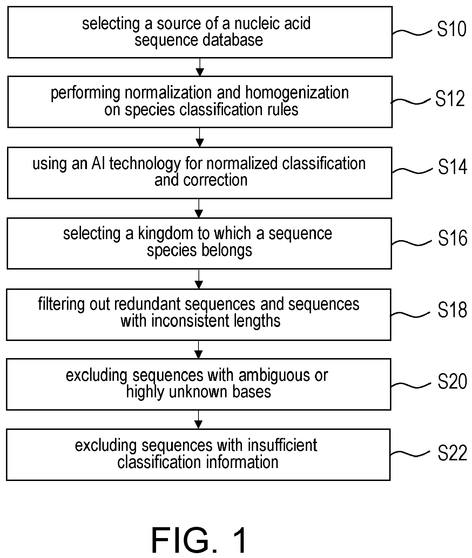

The present disclosure provides a method for constructing a ribosomal RNA database. FIG. 1 is a schematic flowchart of a method for constructing a ribosomal RNA database according to an embodiment of the present disclosure. Hereinafter, the construction method of a ribosomal RNA database according to an embodiment of the present disclosure will be described in detail with reference to FIG. 1.

Please refer to FIG. 1, first, step S10 is performed, and a source of a nucleic acid sequence database is selected. The nucleic acid sequence database may include a native repository database or a value-added database as an initial data source. In the embodiment, the constructed ribosomal RNA database is, for example, a 16S rRNA gene database, and the 16S rRNA gene database will be mainly used as an example in the description below, but the present disclosure is not limited thereto. 16S rRNA is an important component of prokaryotic ribosomal small subunits, which contains conserved regions and 9 highly variable regions. Many studies show that 16S rRNA is highly conserved among different species of bacteria, which means that even if genetic variation occurs to a single species, its 16S rRNA sequence is not easily changed. Therefore, 16S rRNA sequence is very suitable for identifying bacterial and archaeal species.

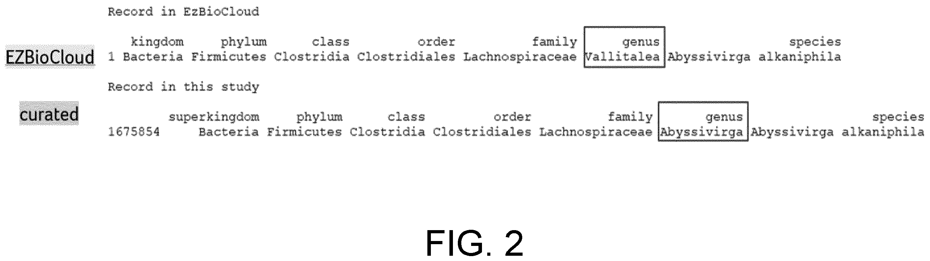

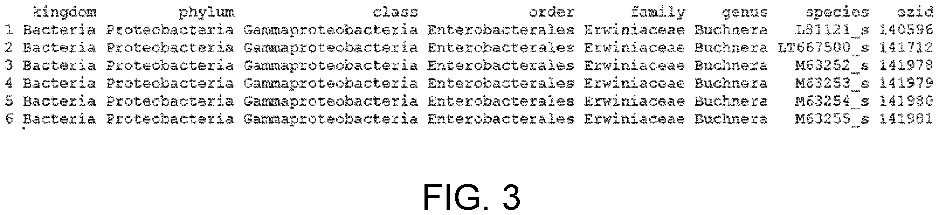

Next, please continue to refer to FIG. 1, and step S12 is carried out to perform normalization and homogenization on species classification rules. In terms of normalization, classification of species is typically based on the classification rules established by Carl Linnaeus. Over years of evolution of the rules, the hierarchies of the classification rules are mainly divided into seven hierarchies: "kingdom, phylum, class, order, family, genus and species". All sequence classification information may be normalized by using this seventh-order classification nomenclature to form a classification hierarchy relation table. In terms of homogenization, the process is performed mainly to homogenize the nomenclature across databases, and correct the wrong information in the source database simultaneously. More specifically, the homogenization process may include, for example, the following two methods. FIG. 2 and FIG. 3 are schematic diagrams of a homogenization method in the construction method of a ribosomal RNA database according to an embodiment of the present disclosure. The first method is, for example, find out information of other hierarchy in the classification hierarchy relation table based on the species name in the nucleic acid sequence database. Please refer to FIG. 2, for example, the species (Abyssivirga alkaniphila) in EZBiocloud is utilized to correspond to the classification hierarchy relation table, and the correction result is shown in curated (please refer to the box marked in red). As for the second method, please refer to FIG. 3. For example, a serial number is used (ID in the species field, e.g. L81121 in FIG. 3) as a search target for comparison with a database that stores serial numbers based on the serial number of species in the nucleic acid sequence database. After the species name of the serial number is found, the information of other hierarchy may be found from the classification hierarchy relation table.

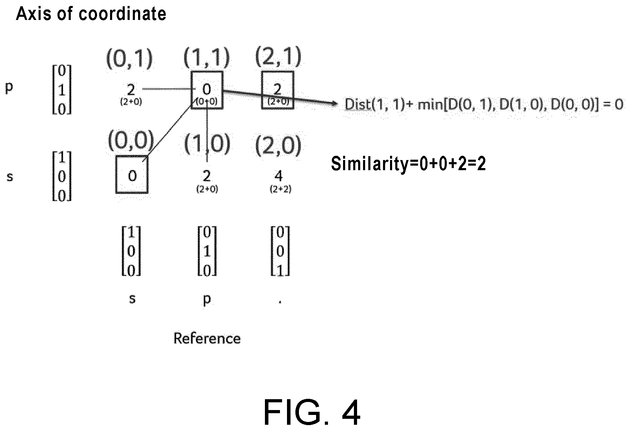

Next, please further refer to FIG. 1 and proceed to step S14. AI technology is used for normalized classification and correction. Comparisons are made mainly based on species hierarchy. In comparison, pairwise comparison is required to confirm that there is no duplication of sequence classification information in the data. There are two comparison methods below. In the first method, for example, a punctuation is replaced with a fixed symbol, and the punctuation is, for example, space, ".", "-" or "/", and the fixed symbol is "_". For example: "Sinorhizobium sp. R-25067" is replaced with "Sinorhizobium_sp_R_25067". Comparison is made according to the adjusted string, and the string is restored to original text after comparison. Since the punctuation may carry other meanings, such as: "sp." refers to one or more species without specifying the exact species, the process may filter out bacterial strains with repeated sequence classification information; for example, "Sinorhizobium sp. R-25067" and "Sinorhizobium sp. R-25067." FIG. 4 is a schematic diagram of performing normalized classification and correction using AI technology in the method for constructing a ribosomal RNA database according to an embodiment of the present disclosure. Please refer to FIG. 4. In the second method, for example, a dynamic time wrapping (DTW) algorithm is adopted. The DTW algorithm is a method through which the text dynamic distance between two strings is compared and the similarity between two characters is measured. Based on a given similarity threshold value, it is possible to determine that the bacterial strains with closer similarity are more likely to belong to the same type of bacteria strains. As such, the problems of similar characters and pinyin or redundant punctuation may be solved. In addition, the method of calculating distance between characters may be Manhattan distance, and the DTW similarity formula is D(i, j)=Dist(i, j)+min[D(i-1, j), D(i, j -1), D(i-1, j-1)]. For example, please refer to FIG. 4. In calculating the similarity between "sp" and "sp.", after converting the text into a matrix, the Manhattan distance is adopted to calculate the distance between characters, and a sum of the minimum distance between each character in Reference is calculated as an index to measure the distances between pairs. After calculation, the similarity between "sp" and "sp." is 2.

Then, please continue to refer to FIG. 1, and proceed to step S16. The kingdom to which the sequence species belongs is selected. In this embodiment, the constructed ribosomal RNA database is, for example, a 16S rRNA gene database. Since 16S rRNA only exists in the Archaea kingdom and Bacteria kingdom, firstly, the sequences belonging to Archaea kingdom and Bacteria kingdom are selected, and other kingdoms or sequences where the kingdom name is mistakenly named as Bacteria or Archaea are excluded. For example,
"Eukaryota;Archaeplastida; Chloroplastida; Chlorophyta; Chlorophyceae; Sphaeropleales; Monoraphidium;Monoraphidium" belong to Eukaryota.

Thereafter, please continue to refer to FIG. 1, and proceed to step S18. Redundant sequences and sequences with inconsistent lengths are filtered out. In terms of filtering out redundant sequences, bacterial strains might contain one or more sets of 16S rRNAs with the same sequence. Due to the high degree of conservation of 16S rRNAs, different subtypes of the same species might have exactly the same sequences. When the sequence contains the same species sequence with 100% identical conditions, it is regarded that the sequence is a redundant sequence and should be filtered out. In terms of sequences with inconsistent lengths, the full length of 16S rRNA is about 1600 bases. Studies show that it is necessary to use sequences covering 9 variable regions in order to accurately identify bacterial strains in the hierarchy of species. If the sequence length is too short, the sequence range for identification is insufficient, which might lead to misclassification of species. If the sequence is too long, it means that the sequence contains two or more sets of 16S rRNA, and other genes might be mixed between the 16S rRNAs, which will also affect the accuracy of species classification. Exclusion conditions for length of sequences are, for example, defined as sequences with shorter than 1200 bases or more than 1800 bases in length.

Next, please continue to refer to FIG. 1, and proceed to step S20. The sequences with ambiguous or highly unknown bases are excluded. 16S rRNA is highly conserved among species, and therefore is highly discriminative among species. Within the classification units of the species hierarchy, the degree of difference between sequences of the same category is generally 1% to 1.3%. If the difference rate between the sequence bases is too high, the sequence will be classified in different species hierarchy. If the unknown bases (not A, T, C, G) contained in the sequence might be identified as sequence errors in the calculation process, the sequence error rate is too high, and it is easy to cause subsequent comparison errors, resulting in the sequence being misclassified in the species hierarchy. In order to exclude an excessively high difference rate and retain the flexibility to allow sequence errors due to sequencing, a threshold is set to exclude unknown bases that exceed the threshold. For example, sequences with 0.5% or more of unknown bases (not A, T, C, G) are excluded first. For example, if an N character is carried in the sequence, it means that the sequence of the site is unknown.

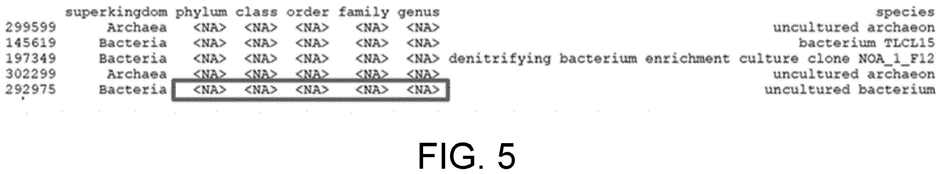

Finally, please continue to refer to FIG. 1 and proceed to step S22. Sequences with insufficient classification information are excluded. FIG. 5 is a schematic diagram of excluding sequences with insufficient classification information in the method for constructing a ribosomal RNA database according to an embodiment of the present disclosure. Because there are still a large number of species that cannot be separated and cultured in the laboratory, their names will be assigned as uncultured bacterium / uncultured archaeoote. Such sequences cannot provide effective information for species identification, and therefore their species names are uncultured bacterium / uncultured archaeoote, and sequences with no information in the first five classification hierarchies of the species are excluded, as the box marked in red in FIG. 5.

To sum up, the present disclosure provides a method for constructing a ribosomal RNA database, including multiple filtering processes and ensuring the integrity and interpretability of the sequence species classification hierarchy. It is expected to increase the accuracy for processing ribosomal RNA sequence data analysis, so as to improve the prediction accuracy of microbial phase. By using the construction method of a ribosomal RNA database of the present disclosure, a high-quality and high-accuracy ribosomal RNA database may be established, and the ribosomal RNA database may be used for cross-comparison with the data adopting the standard classification nomenclature, and the method of the disclosure may be directly applied to the analytical process of microbial phase.

More specifically, the construction method of a ribosomal RNA database of the present disclosure may ensure that the most important sequence names are not likely to be misspelt or mistaken based on the ribosomal RNA database that is normalized and homogenized while having cross-database comparability. After the database is filtered by setting multiple conditions, the amount of data is considerably reduced, which helps to reduce the calculation time and the database is easier to maintain. The constructed ribosomal RNA database is suitable for use as a standard database for comparison, for comparing with unknown sequences obtained by researchers, so the sequence information in the database must be representative and informative. Therefore, exclusion of sequences with a large number of ambiguous or highly unknown bases may improve the interpretability of analysis results.

## Claims

1. A method for constructing a ribosomal RNA database, comprising:
(S10): selecting a source of a nucleic acid sequence database;
(S12): performing normalization and homogenization on species classification rules;
(S14): using an AI technology for normalized classification and correction;
(S16): selecting a kingdom to which a sequence species belongs;
(S18): filtering out redundant sequences and sequences with inconsistent lengths;
(S20): setting a threshold for unknown bases other than A, T, C or G, and excluding the unknown bases that exceed the threshold; and
(S22): excluding sequences with insufficient classification information.

2. The method for constructing the ribosomal RNA database according to claim 1, wherein the nucleic acid sequence database comprises a native repository database or a value-added database.

3. The method for constructing the ribosomal RNA database according to claim 1 or 2, wherein the ribosomal RNA database comprises a 16S rRNA gene database.

4. The method for constructing the ribosomal RNA database according to one of the preceding claims, wherein a seventh-order nomenclature is used for normalization to form a hierarchy relation table, and hierarchies defined in the seventh-order nomenclature comprise kingdom, phylum, class, order, family, genus, and species.

5. The method for constructing the ribosomal RNA database according to claim 4, wherein the method for homogenization comprises finding out information of other hierarchy in the classification hierarchy relation table based on species names in the nucleic acid sequence database, or using a serial number as a search target for comparison with a database that stores the serial numbers based on a serial number of a species in the nucleic acid sequence database, after a species name of the serial number is found, the information of other hierarchy is found from the classification hierarchy relation table.

6. The method for constructing the ribosomal RNA database according to claim 5, wherein the step (S 10, S12, S14, S16, S18, S20, S22) of using the AI technology to perform normalized classification and naming comprises performing a comparison according to a species hierarchy, so as to confirm that there is no repetition in sequence classification information.

7. The method for constructing the ribosomal RNA database according to one of the claims 3-6, wherein the step (S10, S12, S14, S16, S18, S20, S22) of selecting the kingdom to which the sequence species belongs comprises selecting sequences belonging to a kingdom of Archaea and a kingdom of Bacteria directed at the 16S rRNA gene database, and excluding other kingdoms or sequences where a kingdom name is mistakenly named as Archaea or Bacteria.

8. The method for constructing the ribosomal RNA database according to one of the claims 3-7, wherein in the 16S rRNA gene database, when a sequence contains the same species sequence with 100% identical conditions, the sequence is a redundant sequence.

9. The method for constructing the ribosomal RNA database according to one of the claims 3-8, wherein in the 16S rRNA gene database, sequences with inconsistent lengths are those that are shorter than 1200 bases or longer than 1800 bases in length.
